# EUROPEAN PATENT SPECIFICATION

(11) **EP 0 979 110 B1**
(45) Date of publication and mention of the grant of the patent: **25.07.2001**
(21) Application number: 98913051.3
(22) Date of filing: 24.03.1998
(51) Int. Cl.: A61M 1/36

(54) **WEIGHT MEASUREMENT OF BLOOD VOLUME IN SOFT-SHELL VENOUS RESERVOIRS**
GWICHTSBESTIMMUNG VON BLUTVOLUMEN IN WEICHSCHALIGEN BEHÄLTERN FÜR VENÖSES BLUT
MESURE PONDERALE DU VOLUME SANGUIN DANS UN RECIPIENT VEINEUX A PAROI SOUPLE

(30) Priority: 29.04.1997 US 840685
(43) Date of publication of application: 16.02.2000
(73) Proprietor: Medtronic Inc., Minneapolis, MN 55432 (US)
(72) Inventor: VAN DRIEL, Michael, R., Fountain Valley, CA 92708 (US); GRAY, Darren, S., Grand Junction, CO 81506-1919 (US); LAM, Victor, C., H., Honolulu, HI 96817 (US); UYENO, Jill, E., Honolulu, HI 96817 (US); WONG, Yu-Tung, Huntington Beach, CA 92649-3027 (US)
(74) Representative: Hughes, Andrea Michelle
(86) International application number: PCT/US98/05664
(87) International publication number: WO 98/48866

(56) References cited:
- EP-A- 0 329 786
- EP-A- 0 766 974
- US-A- 4 243 833

## Description

This invention relates to the measurement of the blood volume in a soft-shell venous reservoir of a heart-lung machine, and more particularly to a method and apparatus using variations in the weight of the reservoir to track blood volume variations.

Heart-lung machines conventionally include a venous reservoir which receives the patient's blood at a variable rate during open-heart surgery and releases it at a substantially steady rate to the oxygenation circuit from which it is returned to the patient. In the operation of the heart-lung machine, it is important for the perfusionist to be continuously advised of the exact volume of blood in the reservoir, as this information is needed to maintain the correct diluted blood volume in the patient and to calculate the proper doses of infused drugs.

Rigid hard-shell reservoirs lend themselves well to this purpose because accurate graduations can readily be inscribed on their surface. However, because the volume of the hard-shell reservoir itself is constant, it will discharge potentially lethal air into the blood circuit of the heart-lung machine if it is allowed to become empty.

Collapsible soft-shell reservoirs (i.e. plastic bags) have the advantage of increasing and reducing their volume in accordance with the amount of blood they contain, and they consequently need no airspace that could produce emboli. On the other hand, soft-shell reservoirs, because they are always exactly filled with blood, cannot provide a visible volume indication by way of graduations.

In the past, perfusionists have estimated the blood volume in soft-shell reservoirs by the appearance of the reservoir bag, but this requires experience and is not sufficiently accurate for modern requirements. To remedy this deficiency, it has previously been proposed to position the reservoir bag between two parallel plates which are biased against the bag, and whose distance from each other is indicated by a tape measure. That system, however, is not very accurate and is awkward to observe.

EP-A-0329786 discloses a blood separator in which bags containing component parts of blood may be suspended and weighed.

EP-A-766974 discloses a system in which the blood volume in a reservoir is determined by the use of a projection having a cross-sectional area which decreases towards the bottom, and on which a scale is marked to indicate the blood volume.

According to one aspect, the present invention provides a method of measuring the blood volume in a soft-shell venous reservoir, comprising the steps of:
a) computing the volume of blood in said reservoir; and
b) displaying said computed volume; characterised by
c) weighing said reservoir;
d) sensing the hematocrit of the blood in said reservoir; and
e) computing the volume of blood in said reservoir as a function of weight and hematocrit.

According to another aspect, there is provided a system for measuring the blood volume in a soft-shell venous reservoir, comprising:
a) a scale; and
b) a soft-shell blood reservoir; characterised in that said reservoir is suspended from said scale; and further characterised by
c) a hematocrit sensor so positioned as to sense the hematocrit of the blood in said reservoir;
d) said scale and sensor being arranged to produce first and second signals representative, respectively, of the net weight of the contents of said reservoir and of said hematocrit;
e) computing apparatus receiving said first and second signals and computing therefrom an indication of the volume of blood in said reservoir; and
f) display apparatus connected to said computing apparatus and arranged to display said computed volume.

The present invention thus fills the above-described need by suspending the soft-shell venous reservoir bag from e. g. a strain gauge scale and monitoring its weight as the volume of blood in the bag increases and decreases. Because the weight of a given volume of blood varies with its dilution by saline during surgery (and, for that matter, varies from patient to patient), the present invention takes advantage of the fact that the weight of blood per unit volume is a known function of hematocrit. Hematocrit in turn is readily measurable by conventional optical sensors which are in common use in cardio-surgical environments. By electronically combining hematocrit and weight indications, a very precise digital readout of blood volume in the soft-shell reservoir can be continuously obtained, regardless of the shape of the reservoir. Furthermore, the readout in this method is unaffected by any entrapped air.

Preferred embodiments will now be described, by way of example only, with reference to the drawings.

Fig. 1 is a schematic side view of a system according to this invention; and

Fig. 2 is a schematic front view of the system of Fig. 1.

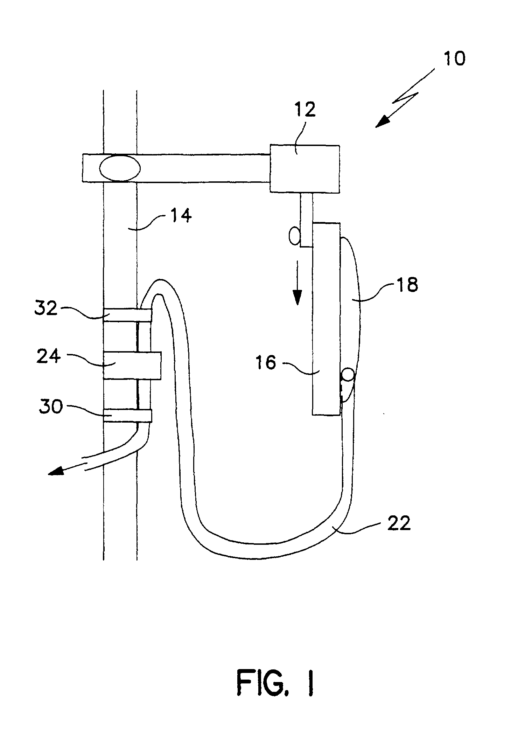

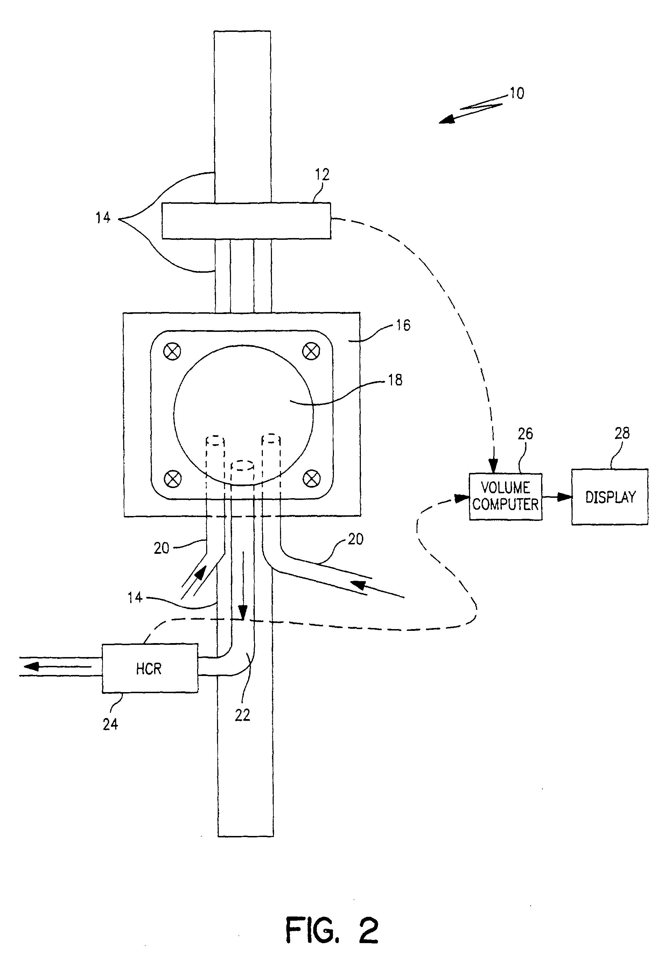

Figs. 1 and 2 schematically illustrate a volume measuring system 10 according to this invention. A conventional strain gauge scale providing an electronic weight indication is mounted on a conventional IV pole 14. Suspended from the scale 12 is a frame or backplate 16 which supports a soft-shell venous reservoir 18 equipped with blood inlets 20 and a blood outlet 22. The blood outlet line 22 is routed through a conventional hematocrit sensor 24 such as Medtronic, Inc.'s Biotrend sensor which produces in real time a continuous hematocrit indication of the blood flowing through line 22.

The weight indication from scale 12 and the hematocrit indication from sensor 24 are mathematically combined in accordance with the known relationship of hematocrit to blood density in a microprocessor or volume computer 26 which continuously computes blood volume.

For the purposes of this computation, hematocrit can be directly related to density. The relationship of formula (1) below has been found to be true for regular undiluted blood as well as for blood diluted with saline; the latter relationship was determined experimentally, using data on density, volume, and mass. For blood diluted with saline, the following linear relationship was obtained:$\text{D=m Hct + b}$ where D is the density, Hct is the hematocrit, and m and b are constants. Using the fact that volume=mass/density, equation (1) was manipulated to equation (2)$\text{V(ml)= M(g)/m / Hct+b/m}$ where V is the volume and M is the net mass or weight of the contents of the reservoir 18. Using experimental data, m and b were determined for formula (1) to provide${\text{D(g/cm}}^{\text{3}} {\text{)=7.2 x 10}}^{\text{-4}} \text{Hct+1.005}$

Thus, the blood volume in the bag 18 will be${\text{V(ml) = M(g) / 7.2 x 10}}^{\text{-4}} \text{Hct + 1.005}$ Equation (4) yields the volume to within 1% and .5% for low/normal and high hematocrit readings, respectively.

The Biotrend itself measures hematocrit to within ±3 points. This translates to a ±0.2% maximum error in the predicted density. When these two errors are added in quadrature, the error is still ±1.00%. This overall error corresponds to 20 ml for 2 1 of blood volume, which is ample accuracy for surgical purposes.

The blood volume computed as outlined above can readily be digitally displayed on a display 28.

The system 10 is calibrated prior to each use by filling the blood circuit and reservoir 18 with a known amount of saline solution (which has a hematocrit of zero), and setting the display 28 to indicate the known volume of saline in the reservoir 18 (i.e. the introduced volume of saline less the volume of the blood circuit lines). Then, as the saline solution is replaced by blood during surgery, changes in weight and hematocrit will increase or decrease that original reading with an accuracy more than sufficient for any surgical need.

Regarding an additional aspect of the invention illustrated in Fig. 1, the peristaltic action of the heart-lung machine's blood pump (not shown) causes pulsating vibrations which are transmitted through the line 22 and can cause inaccuracies or fluctuations in the weight indication of scale 12. Inasmuch as this artifact is generally sinusoidal in nature, it can be eliminated by conventional electronic means, but a simpler way in accordance with this invention is to clamp the line 22 to the IV pole 14 at spaced locations by a pair of clamps 30, 32. Sufficient slack should be left in line 22 between clamp 32 and reservoir 18 to prevent any movement of reservoir 18 from significantly affecting the weight measurement of scale 12. The clamping of line 22 does not fully eliminate the vibration transmitted by the blood pump through the blood, but it damps it sufficiently to maintain a satisfactory accuracy of the volume reading.

It will be noted that unlike other methods of measuring the blood volume in a soft-shell venous reservoir, the above-described method is not subject to volume errors caused by entrapped air, because it measures the blood volume itself rather than the volume of the reservoir bag. Consequently, bleeding the air from the bag 18 is unnecessary.

## Claims

1. A method of measuring the blood volume in a soft-shell venous reservoir (18), comprising the steps of:
a) computing the volume of blood in said reservoir (18); and
b) displaying said computed volume; characterised by
c) weighing said reservoir (18) ;
d) sensing the hematocrit of the blood in said reservoir (18); and
e) computing the volume of blood in said reservoir as a function of weight and hematocrit.

2. The method of Claim 1, in which said volume of blood is computed substantially as${\text{V(ml) = M(g) / 7.2 x 10}}^{\text{-4}} \text{Hct + 1.005}$ wherein V is the blood volume in said reservoir (18), M is the net weight of the contents of said reservoir, and Hct is said hematocrit.

3. A system for measuring the blood volume in a soft-shell venous reservoir, comprising:
a) a scale (12); and
b) a soft-shell blood reservoir (18); characterised in that said reservoir (18)is suspended from said scale; and further characterised by
c) a hematocrit sensor (24) so positioned as to sense the hematocrit of the blood in said reservoir;
d) said scale and sensor being arranged to produce first and second signals representative, respectively, of the net weight of the contents of said reservoir and of said hematocrit;
e) computing apparatus (26) receiving said first and second signals and computing therefrom an indication of the volume of -blood in said reservoir; and
f) display apparatus (28) connected to said computing apparatus and arranged to display said computed volume.

4. The system of Claim 3, in which said volume of blood is computed substantially as${\text{V(ml) = M(g) / 7.2 x 10}}^{\text{-4}} \text{Hct + 1.005}$ wherein V is the blood volume in said reservoir, M is the net weight of the contents of said reservoir, and Hct is said hematocrit.

5. The system of Claim 3 or 4, further comprising:
g) an outlet conduit (22) arranged to convey blood pumped out of said reservoir (18); and
h) a fixed support, said conduit being clamped to said support, with a slack portion of said outlet conduit between the clamping point (30, 32) and said reservoir, so as to damp the transmission of cyclical pumping vibrations to said reservoir (18).

6. The system of Claim 5, in which said hematocrit sensor (24) is positioned to sense the hematocrit of blood passing through said outlet conduit (22).

## Patentansprüche

1. Verfahren zum Messen des Blutvolumens in einem weichschaligen Behälter (18) für venöses Blut, mit folgenden Schritten:
a) Berechnung des Blutvolumens in dem Behälter (18); und
b) Anzeige des berechneten Volumens; gekennzeichnet durch
c) Wiegen des Behälters (18);
d) Abfühlen bzw. Sensieren des Hämatokrit des Blutes in dem Behälter (18); und
e) Berechnung des Blutvolumens in dem Behälter als Funktion des Gewichts und des Hämatokrits.

2. Verfahren nach Anspruch 1, bei dem das Blutvolumen im wesentlichen berechnet wird als${\text{V(ml) = M(g)/7,2 x 10}}^{\text{-4}} \text{Hct + 1,005,}$ wobei V das Blutvolumen in dem Behälter (18), M das Nettogewicht des Inhalts des Behälters, und Hct das Hämatokrit ist.

3. System zur Messung des Blutvolumens in einem weichschaligen Behälter für venöses Blut, mit
a) einer Wiegeeinrichtung (12); und
b) einem weichschaligen Behälter (18) für Blut;
dadurch gekennzeichnet, daß der Behälter (18) an der Wiegeeinrichtung aufgehängt ist; und ferner gekennzeichnet durch
c) einen Hämatokrit-Sensor, der derart positioniert ist, daß er das Hämatokrit des Blutes in dem Behälter abfühlt bzw. sensiert;
d) wobei die Wiegeeinrichtung und der Sensor derart ausgebildet sind, daß sie erste bzw. zweite Signale erzeugen, welche das Nettogewicht des Inhalts des Behälters bzw. das Hämatokrit darstellen;
e) eine Recheneinrichtung (26), welche die ersten und zweiten Signale empfängt und aus diesen eine Angabe bezüglich des Blutvolumens in dem Behälter berechnet; und
f) eine Anzeigevorrichtung (28), die mit der Recheneinrichtung verbunden ist und zur Anzeige des berechneten Volumens ausgebildet ist.

4. System nach Anspruch 3, bei welchem das Blutvolumen im wesentlichen berechnet wird als${\text{V(ml) = M(g) /7,2 × 10}}^{\text{-4}} \text{Hct + 1,005,}$ wobei V das Blutvolumen in dem Behälter, M das Nettogewicht des Inhalts des Behälters, und Hct das Hämatokrit ist.

5. System nach einem der Ansprüche 3 oder 4, ferner mit
g) einem Auslaßkanal (22), der zum Transport von aus dem Behälter (18) gepumptem Blut ausgelegt ist; und
h) einer festen Halterung, wobei der Kanal an die Halterung angeklemmt ist, wobei ein lockerer bzw. schlaffer Abschnitt des Auslaßkanals zwischen dem Anklemmpunkt (30, 32) und dem Behälter ausgebildet ist, so daß die Übertragung von zyklischen Pumpvibrationen auf den Behälter (18) gedämpft wird.

6. System nach Anspruch 5, bei welchem der Hämatokrit-Sensor (24) zum Abfühlen des Hämatokrit des Blutes, welches durch den Auslaßkanal (22) passiert, positioniert ist.

## Revendications

1. Procédé de mesure du volume sanguin dans un réservoir veineux à enveloppe souple (18), comprenant les étapes consistant à :
a) calculer le volume de sang dans ledit réservoir (18) ; et
b) afficher ledit volume calculé ; caractérisé par le fait de
c) peser ledit réservoir (18) ;
d) détecter l'hématocrite du sang dans ledit réservoir (18) ; et
e) calculer le volume de sang dans ledit réservoir en fonction du poids et de l'hématocrite.

2. Procédé selon la revendication 1, dans lequel ledit volume de sang est calculé substantiellement par${\text{V(ml) = M(g) / 7,2 x 10}}^{\text{-4}} \text{Hct + 1,005}$ où V est le volume sanguin dans ledit réservoir (18), M est le poids net du contenu dudit réservoir, et Hct est ledit hématocrite.

3. Système de mesure du volume sanguin dans un réservoir veineux à enveloppe souple, comprenant :
a) une balance (12) ; et
b) un réservoir de sang à enveloppe souple (18) ;
caractérisé en ce que ledit réservoir (18) est suspendu à ladite balance ; et caractérisé en outre par
c) un détecteur d'hématocrite (24) positionné de manière à détecter l'hématocrite du sang dans ledit réservoir ;
d) ladite balance et ledit détecteur étant disposés de manière à produire des premier et deuxième signaux représentatifs respectivement du poids net du contenu dudit réservoir et dudit hématocrite ;
e) un dispositif calculateur (26) recevant lesdits premier et deuxième signaux et calculant à partir de ceux-ci une indication du volume de sang dans ledit réservoir ; et
f) un dispositif d'affichage (28) connecté audit dispositif calculateur et prévu pour afficher ledit volume calculé.

4. Système selon la revendication 3, dans lequel ledit volume de sang est calculé substantiellement par${\text{V(ml) = M(g) / 7,2 x 10}}^{\text{-4}} \text{Hct + 1,005}$ où V est le volume sanguin dans ledit réservoir, M est le poids net du contenu dudit réservoir, et Hct est ledit hématocrite.

5. Système selon la revendication 3 ou 4, comprenant en outre :
g) une conduite de sortie (22) prévue pour transporter le sang pompé hors dudit réservoir (18) ; et
h) un support fixe, ladite conduite étant fixée audit support, avec une portion lâche de ladite conduite de sortie entre le point de fixation (30, 32) et ledit réservoir, de manière à amortir la transmission des vibrations de pompage cycliques audit réservoir (18).

6. Système selon la revendication 5, dans lequel ledit détecteur d'hématocrite (24) est positionné de manière à détecter l'hématocrite du sang passant à travers ladite conduite de sortie (22).
